Europäisches Patentamt
European Patent Office
Office européen des brevets

(19)

(11) Publication number: **0 522 506 A2**

(12) **EUROPEAN PATENT APPLICATION**

(21) Application number: **92111500.2**

(22) Date of filing: **07.07.92**

(51) Int. Cl.5: **A61K 7/00**, C07C 69/24, C07C 69/52

(30) Priority: **09.07.91 JP 168196/91**

(43) Date of publication of application: **13.01.93 Bulletin 93/02**

(84) Designated Contracting States: **DE FR GB**

(71) Applicant: **KAO CORPORATION**
**1-14-10, Nihonbashikayaba-cho**
**Chuo-ku Tokyo(JP)**

(72) Inventor: **Ohashi, Yukihiro**
**30-19, Koedomachi**
**Utsunomiya-shi, Tochigi(JP)**
Inventor: **Kawamata, Akira**
**3-1-17-707, Hanabusa**
**Utsunomiya-shi, Tochigi(JP)**

(74) Representative: **Wächtershäuser, Günter, Dr.**
**Tal 29**
**W-8000 München 2(DE)**

(54) **Ester compound and cosmetic preparation comprising the same.**

(57) Ester compounds represented by the following general formula (I):

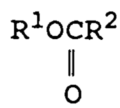

(I)

wherein $R^1$ is a group

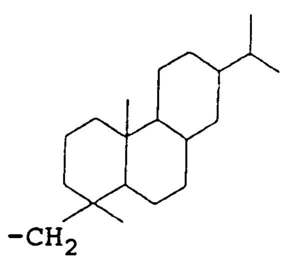

,

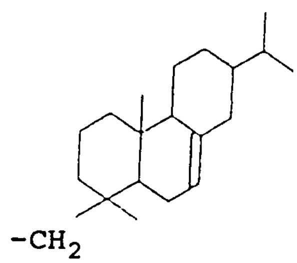

,

EP 0 522 506 A2

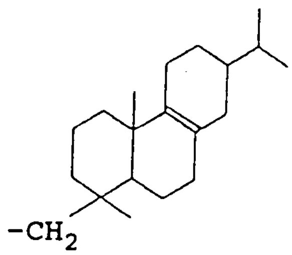 or 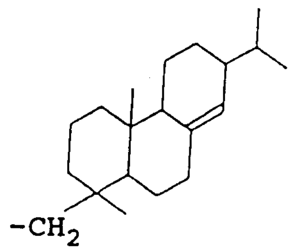 ,

and $R^2$ is a straight-chain or branched, saturated or unsaturated hydrocarbon group having 7-25 carbon atoms, may be formulated into cosmetic compositions, which are nonirritating, have good coating and protective properties, and are stable for long periods of time.

## BACKGROUND OF THE INVENTION

### Field of the Invention:

The present invention relates to novel ester compounds and cosmetic compositions comprising the same, and more specifically to novel ester compounds which are liquid at room temperature, stable, are scarcely irritating to the skin, exhibit excellent coating ability, adhesive properties and protective effects for the skin and hair, and maintain these effects for long periods of time, and cosmetic compositions containing such an ester compound.

### Description of the Background:

The outermost layers of the skin and hair contain a moderate amount of water. This water content is essential for maintaining the elasticity, softness, protective function and the like of the skin and hair. Therefore, when the water content decreases, the skin, for example, can deteriorate to the state of so-called dry skin, and loses its elasticity and protective function, causing various problems. On the other hand, sebum is known to prevent unnecessary evapotranspiration of water in the outermost layers of the skin and hair and hence play an important role in avoiding the above-described problems.

Accordingly, it has heretofore been a common practice to use various oily substances led by squalane, which is a constituent of the skin, by incorporating them into cosmetic preparations and the like, in order to prevent excess dryness of the skin or hair.

However, the conventional oily substances such as squalane suffer from the drawbacks that they are insufficient in coating ability and adhesive properties to the skin and hair, and can neither provide a satisfactory protective effect nor maintain such an effect for a sufficient period of time.

Accordingly, there remains a need for cosmetic compositions which exhibit a low skin-irritating tendency, are excellent in coating ability, adhesive properties and protective effects for the skin and hair, and can maintain these effects for a longer period of time.

## SUMMARY OF THE INVENTION

Accordingly, it is one object of the present invention to provide novel ester compounds which are liquid at room temperature.

It is another object of the present invention to provide novel ester compounds which do not irritate the skin, have excellent coating, adhesive, and protective properties for the skin and hair and maintain the effects for a long period of time.

It is another object of the present invention to provide cosmetic compositions which contain such ester compounds.

These and other objects, which will become apparent during the following detailed description, have been achieved by the inventors' discovery that the novel ester compounds represented by the general formula (I), which will be described subsequently, are excellent in coating ability, adhesive properties and protective effects for the skin and hair, and can maintain these effects for long periods of time. Further, these ester compounds cause scarcely any irritation to the skin and are high in safety, and the use of such ester compounds can provide cosmetic compositions which overcome the above-mentioned problems.

Thus, in a first aspect of the present invention, there is provided an ester compound represented by the following general formula (I):

$$R^{1}O\underset{\underset{O}{\|}}{C}R^{2} \qquad (I)$$

wherein $R^1$ is selected from the group consisting of

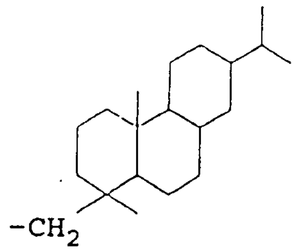
,
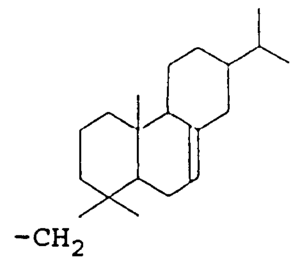
,

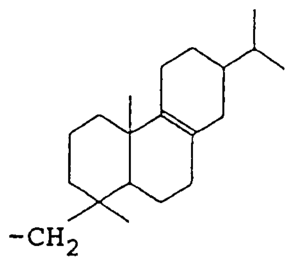
and
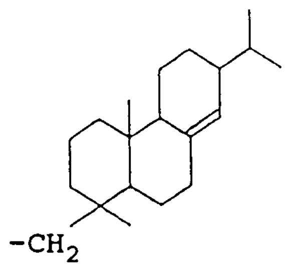
,

and $R^2$ is a straight-chain or branched, saturated or unsaturated hydrocarbon group having 7-25 carbon atoms.

In another aspect of this invention, there is also provided a cosmetic composition comprising one or more of the ester compounds represented by the general formula (I).

The ester compounds according to this invention are liquid somewhat high in viscosity at room temperature, chemically stable, cause scarcely any irritation to the skin, are excellent in coating ability, adhesive properties and protective effects for the skin and hair, and can maintain these effects for a long period of time.

Cosmetic preparations containing at least one ester compound of this invention can enhance the water-retaining ability of the skin and hair, thereby imparting good elasticity and softness thereto, improving the protective function and avoiding the condition of dry skin and the like.

## DETAILED DESCRIPTION OF THE PREFERRED EMBODIMENTS

In the general formula (I), $R^2$ is a straight-chain or branched, saturated or unsaturated hydrocarbon group having 7-25 carbon atoms, preferably 12-20 carbon atoms. However, a straight-chain or branched alkyl or alkenyl group having 7-25 carbon atoms, preferably 12-20 carbon atoms, is preferred.

The ester compounds according to the present invention are novel compounds, but can be prepared with ease in accordance with the conventionally-known ester interchange reactions. Namely, the ester derivative (I) of this invention can be obtained by (a) reacting an abietyl alcohol (II) with a carboxylic acid (III) in the presence of an acid catalyst in accordance with the following reaction formula (A); (b) using the abietyl alcohol (II) and an lower alcohol ester (IV) of a carboxylic acid to conduct an ester exchange (transesterification) reaction in the presence of a base catalyst in accordance with the following reaction formula (B); or (c) reacting the abietyl alcohol (II) with a carboxylic acid chloride (V) in accordance with the following reaction formula (C).

(A) 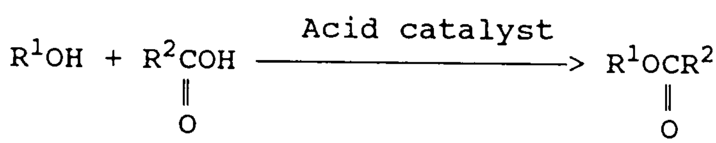


(II) (III) (I)

$$\text{(B)}\quad R^1OH + R^2\underset{\substack{\| \\ O}}{C}OR^3 \xrightarrow{\text{Base catalyst}} R^1O\underset{\substack{\| \\ O}}{C}R^2$$

(II) (IV) (I)

$$\text{(C)}\quad R^1OH + R^2\underset{\substack{\| \\ O}}{C}OCl \longrightarrow R^1O\underset{\substack{\| \\ O}}{C}R^2$$

(II) (V) (I)

wherein $R^1$ and $R^2$ have the same meaning as defined above, and $R^3$ means a lower alkyl group.

In the above-described preparation processes, the abietyl alcohol (II) used as a raw material can be prepared from rosin (colophonium) or abietic acid obtained by purifying rosin in accordance with a process known per se in the art (for example, U.S. Patent Nos. 2,146,897 and 2,358,235, J. Org. Chem., Vol. 34, 3464 (1969), Mokuzai Gakkai-Shi (Journal of Wood Society), Vol. 27, 649 (1981), etc.).

As illustrative examples of the carboxylic acid (III) used in the above-described process, may be mentioned capric acid, lauric acid, myristic acid, palmitic acid, stearic acid, behenic acid, palmitoleic acid, oleic acid, linoleic acid, linolenic acid, arachidonic acid, erucic acid, hexadecanoic acid, 2-ethylhexanoic acid, 2-hexyldecanoic acid, 2-heptylundecanoic acid, 5,7,7-trimethyl-2-(1,3,3-trimethylbutyl)octanoicacid, methyl-branched isostearic acid, etc.

As illustrative examples of the lower alcohol ester (IV) of a carboxylic acid, may be mentioned methyl esters, ethyl esters, propyl esters, butyl esters, etc. of the above-mentioned carboxylic acids.

Sulfuric acid, benzenesulfonic acid, p-toluenesulfonic acid or the like may be used as the acid catalyst for the esterification in the above-described process, while sodium methoxide, potassium methoxide or the like may be used as the base catalyst for the transesterification.

The thus-obtained ester compounds (I) according to the present invention are stable, cause scarcely any irritation to the skin and have excellent coating ability, adhesive properties and protective effects for the skin and hair, and can hence be used as oily bases for skin and/or hair cosmetics.

The proportion of the ester derivative (I) in the cosmetic composition according to this invention may be suitably determined as necessary for the end application intended. In general, it is however incorporated in a proportion of 0.1-80 wt.% (hereinafter indicated merely by "%"), preferably 1-50%, based on the total weight of the cosmetic composition, in the cosmetic composition.

In the cosmetic compositions according to the present invention, it is possible to incorporate, in addition to the essential ingredient described above, other ingredients employed routinely as cosmetic ingredients, such as a cosmetically acceptable carrier, for example,
higher alcohols having a straight-chain or branched alkyl or alkenyl group; hydrocarbons such as liquid paraffin, vaseline and solid paraffin; lanolin derivatives such as liquid lanolin and lanolin fatty acid; silicone derivatives such as dimethylpolysiloxane, polyether-modified polysiloxane and amino-modified polysiloxane; oils and fats such as esters of higher alcohols with higher fatty acids, higher fatty acids and amidoamines having a long chain alkyl or alkenyl; animal and vegetable oils and fats such as mink oil and olive oil; medicinally-effective ingredients such as antidandruff agents, disinfectants and vitamins; antiseptics such as Parabens; thickeners such as water-soluble polymers; colorants such as dyes and pigments; ultraviolet absorbents; chelating agents; pH regulators; astringents; moisturisers such as propylene glycol, glycerol, carbitol, 3-methyl-1,3-butanediol and saccharides; various kinds of emulsifiers; water; perfume bases; and the like so far as they do not impede the effects of the inventive ester. Further examples and discussion of suitable additional ingredients are found in Kirk-Othmer, Encyclopedia of Chemical Technology, 3rd ed., John Wiley & Sons, New York, Vol. 7, pp. 143-176 (1979), which is incorporated herein by reference.

The cosmetic compositions according to the present invention may be formulated in any forms such as an oil-based, water-based and emulsified types, are particularly suitable for use in skin-care preparations or hair preparations for treating rough skin or hair, for which a water-retaining effect is required, and can be used, for example, as fundamental cosmetics such as oil-in-water or water-in-oil type emulsified cosmetics and oily cosmetics; makeup cosmetics such as lip sticks and foundations; skin cleansing preparations; hair-care products such as hair rinses, hair treatments and hair styling preparations; etc.

Other features of the invention will become apparent in the course of the following descriptions of exemplary embodiments which are given for illustration of the invention and are not intended to be limiting thereof.

## EXAMPLES

### Example 1:

Synthesis of tetrahydroabietyl methyl-branched isostearate:

A 500-ml flask equipped with a vacuum distiller, thermometer and stirrer was charged with 117.0 g (0.4 mol) of tetrahydroabietyl alcohol, 119.4 g (0.4 mol) of methyl methyl-branched isostearate and 0.43 g (0.008 mol) of sodium methoxide. The contents were stirred for 10 hours while the methanol formed was removed by distillation at 100 ° C/20 Torr. After completion of the reaction, hexane was added to the reaction mixture, and the resulting mixture was washed with a saturated saline solution. The solvent was then removed by distillation under reduced pressure, and the residue was purified by chromatography on a silica gel column, thereby obtaining 218.1 g (yield: 97.6%) of the intended tetrahydroabietyl methyl-branched isostearate as a colorless oil.

$^1$H-NMR ($CDCl_3$, $\delta$): 0.55-1.93 (m,66H), 2.32(t,J = 7.3Hz,2H), 3.63(d,J = 10.6Hz,1H), 3.87-(d,J = 10.6Hz,1H).
IR (neat, $cm^{-1}$): 2936, 2860, 1740, 1466, 1384, 1244, 1168.

### Example 2:

Synthesis of tetrahydroabietyl stearate:

Tetrahydroabietyl n-stearate was obtained in an amount of 213.6 g (yield 95.6%) as a colorless oil in the same manner as in Example 1 except that 119.4 g (0.4 mol) of methyl n-stearate was used instead of the methyl methyl-branched isostearate used in Example 1.

$^1$H-NMR ($CDCl_3$, $\delta$): 0.53-2.02(m, 66H), 2.31(t,J = 7.5Hz, 2H), 3.61(d,J = 11Hz,1H), 3.85(d,J = 11Hz,1H).
IR (neat, $cm^{-1}$): 2926, 2854, 1743, 1467, 1386, 1245, 1147.

### Example 3:

Synthesis of tetrahydroabietyl 2-heptylundecanoate:

A 1-liter flask equipped with a stirrer and a liquid-liquid separator having a reflux condenser in its upper part was charged with 117.0 g (0.4 mol) of tetrahydroabietyl alcohol, 136.6 g (0.48 mol) of 2-heptylundecanoic acid, 250 ml of toluene and 2.0 g (0.02 mol) of sulfuric acid. The contents were heated and refluxed with stirring for 24 hours, thereby removing the water formed by distillation. After completion of the reaction, the reaction mixture was washed with an aqueous solution of sodium hydrogen carbonate and then a saturated saline solution. The solvent was then removed by distillation under reduced pressure, and the residue was purified by chromatography on a silica gel column, thereby obtaining 179.1 g (yield: 80.1%) of the intended tetrahydroabietyl 2-hexylundecanoate as a colorless oil.

$^1$H-NMR ($CDCl_3$, $\delta$): 0.52-2.08(m,67H), 2.27-2.40(m,1H), 3.50-3.66(m,1H), 3.80-3.94(m,1H).
IR (neat, $cm^{-1}$): 2932, 2860, 1738, 1466, 1386, 1160.

### Example 4:

Synthesis of tetrahydroabietyl 2-(1,3,3-trimethylbutyl)-5,7,7-trimethyloctanoate:

(a) A 500-ml flask equipped with a reflux condenser, thermometer, dropping funnel, nitrogen inlet tube and stirrer was charged with 184.9 g (0.65 mol) of 2-(1,3,3-trimethylbutyl)-5,7,7-trimethyloctanoic acid. While introducing nitrogen gas, the content was stirred at 40 ° C. To this compound, 85.1 g (0.715 mol) of thionyl chloride was added dropwise over 2 hours with stirring. After completion of the addition, the stirring was continued for an additional 7 hours at 45 ° C. After completion of the reaction, the excess thionyl chloride and toluene were removed by distillation under reduced pressure. The residue was distilled under reduced pressure to obtain 191.1 g (yield: 97.1%) of 2-(1,3,3-trimethylbutyl)-5,7,7-trimethyloctanoic acid chloride.

bp: 105 ° C/0.005 Torr

(b) A 1-liter flask equipped with a reflux condenser, thermometer, dropping funnel, nitrogen inlet tube and stirrer was charged with 117.0 g (0.4 mol) of tetrahydroabiethyl alcohol and 240 ml of toluene. While introducing nitrogen gas, 121.17 g (0.4 mol) of 2-(1,3,3-trimethylbutyl)-5,7,7-trimethyloctanoic acid chloride, which had been obtained in step (a), was added dropwise over 30 minutes with stirring. After completion of the addition, the stirring was continued for an additional 2 hours at 80 ° C. After completion of the reaction, the solvent was removed by distillation under reduced pressure. The residue was then purified by chromatography on a silica gel column, thereby obtaining 208.7 g (yield: 93.3%) of the intended tetrahydroabietyl 2-(1,3,3-trimethylbutyl)-5,7,7-trimethyloctanoate as an oil.

$^1$H-NMR ($CDCl_3$, $\delta$): 0.53-2.02 (m, 67H), 2.16 (bs, 1H), 3.41-4.08 (m,2H).

IR (neat, $cm^{-1}$): 2924, 2872, 1734, 1470, 1386, 1368, 1160.

Example 5:

Synthesis of tetrahydroabietyl 6(z)-hexadecenoate:

(a) A 50-ml flask equipped with a reflux condenser, thermometer, dropping funnel and nitrogen inlet tube was charged with 10.0g (39.3 mmols) of 6(z)-hexadecenoic acid and 5 ml of toluene. While introducing $N_2$ gas, the contents were stirred at 80 ° C. To the contents, a solution of 5.14 g (43.2 mmols) of thionyl chloride in 5 ml of toluene was added dropwise over 30 minutes with stirring. After completion of the addition, the stirring was continued for additional 2 hours at 80 ° C. After completion of the reaction, the excess thionyl chloride and toluene were removed by distillation under reduced pressure to obtain 10.97 g of crude 6(z)-hexadecanoic acid chloride.

(b) A 50-ml flask equipped with a reflux condenser, thermometer, dropping funnel and nitrogen inlet tube was charged with 5.35 g (18.3 mmols) of tetrahydroabietyl alcohol, 2.90 g (36.6 mmols) of pyridine and 10 ml of toluene. The contents were stirred at 100 ° C while introducing nitrogen gas and then a solution of 6(z)-hexadecanoic acid chloride, which had been obtained in step (a), in 5 ml toluene was added dropwise with stirring. After completion of the addition, the stirring was continued for an additional 4 hours at 100 ° C. After completion of the reaction, the reaction mixture was washed with water and the solvent was removed by distillation under reduced pressure. The residue was then purified by chromatography on a silica gel column, thereby obtaining 5.58 g (yield: 57.7%) of the intended tetrahydroabietyl 6(z)-hexadecenoate as a colorless oil.

$^1$H-NMR ($CDCl_3$, $\delta$): 0.53-1.92 (m, 54H), 1.92-2.10 (m,4H), 2.32(t,J = 7.3Hz,2H), 3.62-(d,J = 11.0Hz,1H), 3.85(d,J = 11.0Hz,1H), 5.27-5.43(m,2H).

IR (neat, $cm^{-1}$): 2936, 2860, 1740, 1467, 1386, 1245, 1160.

Example 6:

Synthesis of dihydroabietyl methyl-branched isostearate:

Dihydroabietyl methyl-branched isostearate was obtained in an amount of 205.7 g (yield 92%) in the same manner as in Example 1 except that 116.2 g (0.4 mol) of dihydroabietyl alcohol was used instead of the tetrahydroabietyl alcohol used in Example 1.

$^1$H-NMR ($CDCl_3$, $\delta$): 0.54-2.12(m,64H), 2.32(t,J = 7.3Hz,2H), 3.63(d,J = 10.6Hz,1H), 3.87-(d,J = 10.6Hz,1H).

IR (neat, $cm^{-1}$): 2935, 2860, 1740, 1465, 1385, 1245, 1168.

Example 7:

Test of skin-irritating tendency:

The compounds of the present invention, which had been obtained in Examples 1-6, and liquid paraffin as a control were separately applied once a day to the sides of Hartley white guinea pigs (1 Group: 5 heads), which had been shorn and shaved, for 4 days. On the following day of the completion of the last application, the condition of the skin reaction of each group of guinea pigs was observed and ranked in accordance with the following standard:

0: No reaction occurred;

1: Slight or scattered erythema was observed;
2: Clear erythema was observed;
3: Erythema accompanied by edema was observed;
4: Crust and/or necrosis were observed.

Each score was indicated by an average value. The results are shown in Table 1.

Table 1

| Sample tested | Average score |
|---|---|
| Compound of Example 1 | 0.2 |
| Compound of Example 2 | 0 |
| Compound of Example 3 | 0 |
| Compound of Example 4 | 0.2 |
| Compound of Example 5 | 0.2 |
| Compound of Example 6 | 0.2 |
| Liquid paraffin | 0.2 |

It is apparent from the results shown in Table 1 that the skin-irritating tendency of the ester compounds (I) according to the present invention is comparable with or lower than that of liquid paraffin, which is used as a base material in conventional cosmetic preparations.

Example 8:

A W/O type skin cream having the following composition was prepared.

| | (wt%) |
|---|---|
| Tetrahydroabietyl methyl-branched isostearate (the compound of Example 1) | 1 |
| Lecithin | 0.5 |
| Vaseline | 15.0 |
| Hexadecyl 2-ethylhexanoate | 10.0 |
| Deionized water | Balance |

Example 9:

A W/O type cleansing cream having the following composition was prepared.

| | (wt.%) |
|---|---|
| Tetrahydroabietyl 2-(1,3,3-trimethylbutyl)-5,7,7-trimetyloctanoate (the compound of Example 4) | 1.5 |
| Liquid paraffin | 25.5 |
| Squalane | 25.0 |
| Sorbitan sesquioleate | 0.5 |
| Methyl para-hydroxybenzoate | 0.1 |
| Perfume base | 0.05 |
| Deionized water | Balance |

Example 10:

An O/W type massage cream having the following composition was prepared.

| | (wt.%) |
|---|---|
| Tetrahydroabietyl 2-heptyldodecanoate (the compound of Example 3) | 5.0 |
| Cetyl alcohol | 1.5 |
| Liquid paraffin | 20.0 |
| Isopropyl myristate | 15.0 |
| Vaseline | 10.0 |
| Lecithin | 2.5 |
| Polyoxyethylene sorbitan oleate (20 E.O., E.O. stands for ethylene oxide units) | 1.0 |
| Methyl para-hydroxybenzoate | 0.2 |
| Perfume base | 0.05 |
| Propylene glycol | 10.0 |
| Deionized water | Balance |

Example 11:

An O/W type milky lotion having the following composition was prepared.

| | (wt.%) |
|---|---|
| Tetrahydroabietyl methyl-branched isostearate (the compound of Example 1) | 2.5 |
| Dihydroabietyl methyl-branched isostearate (the compound of Example 6) | 2.5 |
| Polyoxyethylene sorbitan monostearate (20 E.O.) | 1.0 |
| Glycerol monostearate | 1.5 |
| Liquid paraffin | 15.0 |
| Glycerol | 10.0 |
| Propyl para-hydroxybenzoate | 0.1 |
| Deionized water | Balance |

The cosmetic preparations of this invention, which had been obtained in Examples 8-11, were all stable, low in skin-irritating tendency, excellent in coating ability, adhesive properties and protective effects for the skin, and able to maintain these effects for a long period of time.

Obviously, numerous modifications and variations of the present invention are possible in light of the above teachings. It is therefore to be understood that, within the scope of the appended claims, the invention may be practiced otherwise than as specifically described herein.

**Claims**

**1.** An ester compound, having the following formula (I):

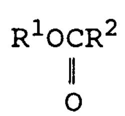

(I)

wherein $R^1$ is selected from the group consisting of

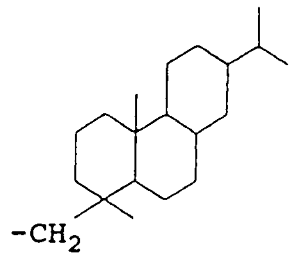
,
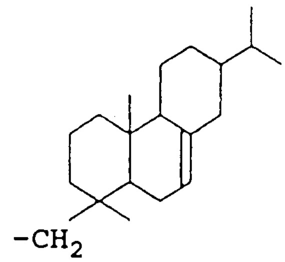
,

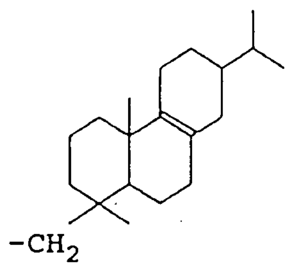
and
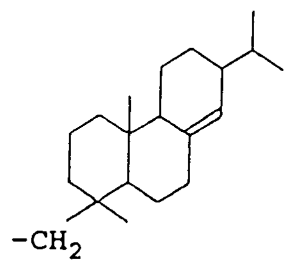
,

and $R^2$ is a straight-chain or branched, saturated or unsaturated hydrocarbon group having 7-25 carbon atoms.

**2.** The ester compound of Claim 1, wherein $R^2$ is a straight-chain or branched alkyl group having 7-25 carbon atoms or a straight-chain or branched or alkenyl group having 7-25 carbon atoms.

**3.** A cosmetic composition, comprising at least one ester compound, having the following formula (I):

$$R^1OCR^2 \quad (\text{with } C{=}O) \qquad (I)$$

wherein $R^1$ is selected from the group consisting of

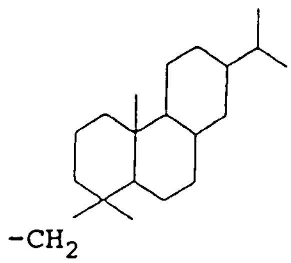 , 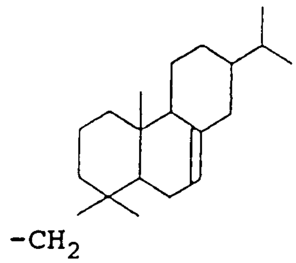 ,

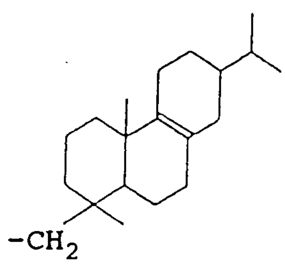 and 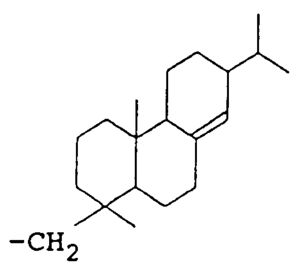 ,

and $R^2$ is a straight-chain or branched, saturated or unsaturated hydrocarbon group having 7-25 carbon atoms; and a cosmetically acceptable carrier.

**4.** The cosmetic composition of Claim 3, wherein said ester compound (I) is incorporated in an amount of 0.1-80 wt.%, based on the total weight of said composition.

**5.** The cosmetic composition of Claim 3, wherein $R^2$ is a straight-chain or branched alkyl group having 7-25 carbon atoms or a straight-chain or branched alkenyl group having 7-25 carbon atoms.